# EUROPEAN PATENT APPLICATION

(11) **EP 1 297 821 A1**
(43) Date of publication of application: **02.04.2003**
(21) Application number: 01308361.3
(22) Date of filing: 01.10.2001
(51) Int. Cl.: A61K 7/48

(54) **Skin treatment compositions**

(71) Applicant: Hive of Beauty (Europe) BVBA, 9140 Temse (BE)
(72) Inventor: Van Steenbergen, Mark Hive of Beauty (Europe) BVBA, 9140 Temse (BE)
(74) Representative: Shaw, Laurence

(57) **Abstract**

A composition for controlling wrinkles in the skin of smokers comprises:
- anti-ageing component
- anti-stress component, and
- anti-oxidant component

## Description

The invention relates to a composition for use in treating skin, especially that of smokers.

It has been realised that smoking has an effect on the skin, in particular it causes the formation of wrinkles and the like. It has been realised that tobacco smoke extract will induce zinc-dependant proteases which degrade dermal collagen and other extracellular matrix molecules. Tobacco smoking also lowers the level of oestrogen in the blood. The nitrogen gases from cigarettes generate carcinogenic nitrosamines in the body tissues. Cigarette smoke is a potentially free radical generator. It is a combination of these factors which cause the skin to become dry and age prematurely in the case of tobacco smokers, especially nicotine addicts. These effects can persist for a period even if the smoker gives up smoking. There is no skin composition available specifically adapted for treating the skin of smokers. It is one object of this invention to provide a skin treatment composition intended to improve the appearance of smokers, both male and female.

According to the invention in one aspect there is provided a skin treatment composition comprising in combination an anti-ageing component, an anti-stress component and an anti-oxidant component.

Preferably each component is made up of a plurality of substances.

Preferably the anti-ageing component is a blend of plant extracts. It is a much preferred feature of the invention that the anti-ageing component is made up of more than one ingredient because there is evidence of a synergistic effect. Preferably, at least one of the ingredients is an extract of Walnuts and the second component is an extract of Beech. Preferably in both cases buds or shoots are used as the source of the component.

The Walnut component is preferably from the Juglans genus, and is extracted by macerating nuts in water at low temperature. The tree is preferably Juglans regia which is the Noix de Genoble walnut tree. The extracts are available commercially from Gattfosse SA of France, as GATULINE AGE DEFENSE.

The Beech extract is preferably from a shoot of the Fagus crenata Blume, Fagus japonika Maxim, Fagus grandifolia, Fagus sylvatica L., Fagus sylvatica L. var. pendula, Fagus sylvatica L. var. purpurea or Fagus orientalis Lipsky, of two or more types. The active ingredient may be extracted using a solvent such as water, ethanol, methanol, propanol, butanol, 1,3-butylene glycol or mixtures thereof. The extracts are available commercially from Gattfossé S.A. of France as GATULINE RC (registered trademark)

It is preferable to include a collagen stimulating peptide, such as tripeptide - 1 as an anti-aging ingredient.

Preferably the composition has a relatively high concentration of the anti-ageing ingredient, from say 10% to about 12% by weight, preferably about 11% by weight.

Anti-stress agents, i.e. anti-skin stress agents, include various vitamins such as such as vitamin A oil, retinol, retinol acetate; vitamin B2s such as riboflavin, riboflavin butyrate, flavinadenine nucleotide; vitamin B6s such as pyridoxine hydrochlorates, pyridoxine dioctanoates; vitamin C such as L-ascorbic acid, L-ascorbate dipalmitate esters, L-ascorbate monopalmitate esters, sodium L-ascorbate-2-sulfate, L-ascorbate phosphate esters, L-ascorbate stearate esters, L-ascorbate-2-glycoside, dipotassium DL- alpha -tocopherol-L-ascorbate phosphate diester; pantothenic acids such as calcium pantothenate, D-pantothenyl alcohol, pantothenyl ethyl ether, acetyl pantothenyl ethyl ether; nicotinic acids such as nicotinic acid, nicotinic acid amide, benzyl nicotinate; vitamin E's such as alpha -tocopherol, tocopherol acetate, DL- alpha -tocopherol nicotinate, DL- alpha -tocopherol succinate; vitamin P, biotin.

Among these vitamins, vitamin C's such as L-ascorbic acid, L-ascorbate dipalmitate esters, L-ascorbate monopalmitate esters, sodium L-ascorbate-2-sulfate, L-ascorbate phosphate esters, L-ascorbate stearate esters, L-ascorbate-2-glycoside, dipotassium DL- alpha -tocopherol-L-ascorbate phosphate retinyl palmitate and tocopherylacetate diester are preferred.

Preferred are acerola extract which is rich in vitamin C, ascorbyl palmitate and tocopherol acetate which is vitamin E. The last two are preferably provided as liposomes because this delays release of the active ingredient.

The concentration of the anti-stress agent ranges from about 2% to about 3% by weight of the composition.

The anti-oxidants may also be a stimulant for the glutothione system, e.g. a protein such as superoxide dismutase, preferably being provided in micro-encapsulated form. The concentration of the anti-oxidant ranges from about 2% to about 3% by weight.

Several of the ingredients can fulfil more than one role. For example, maltodextrin, malpigira glabra, will act as an anti-oxidant, anti-wrinkle, and anti-stress agents.

Several ingredients are available as commercial mixtures. These may be used as long as they do not contain other substances which would interfere with the function of the active ingredients.

Silicones may be present. Examples include dimethyl polysiloxane, methylphenyl polysiloxane, methylhydrogen polysiloxane, cyclic polysiloxanes such as decamethyl polysiloxane, dodecamethyl polysiloxane and tetramethyltetrahydrogen polysiloxane.

Surfactants may be present. These may be anionic, cationic or amphoteric.

As preservatives phenoxyethanol, diazolidinylurea, methylparaben, ethylparaben, butylparaben, etc. may be present.

As metal ion chelates, salts, EDTA, etc. may be present.

Skin conditioners may be present, examples include petrolatum and phospholipids.

Biological additives may be present such as maltodextrin, malpigia glabra, ceratonia silica gum, cyamopsia tetragonoloba gum, pyrus malus and cochlearia armoracia.

Viscosity increasing agents may be present. Examples include hydroxylpropyl starch phosphate, carbomers, Xanthan gum, hydrogenated styrene copolymers and dimethicone/vinyl dimethicone copolymer.

Emollients may be present. Examples include cyclopentasiloxane and hydrogenated polyisobutylene.

Cetearyl aclohol and acrylate crosspolymers may be present as emulsion stabilisers.

A humectant such as glycerin may be present; so too may a pH adjuster such as triethanolamine.

As natural water-soluble polymers, plant base polymers such as gum arabic, tragacanth gum, galactan, guar gum, carob gum, karaya gum, carragheein, pectin, agar, quince seed (Marumero), algae colloid (seaweed extract), starch (rice, corn, potato, wheat), glycyrrhinic acid; microorganism base polymers such as xanthane gum, dextran, succinoglutan, pullulan; animal base polymers such as collagen, caseine, albumin, gelatin; etc. may be present.

As water-soluble polymers, starch base polymers such as carboxymethyl starch, methylhydroxpropyl starch; cellulose base polymers such as methyl cellulose, nitro cellulose, ethyl cellulose, methylhydroxypropyl cellulose, hydroxyethyl cellulose, sodium cellulose sulfate, hydroxypropyl cellulose, sodium carboxymethyl cellulose (CMC), crystalline cellulose, cellulose powder; alginate base polymers such as sodium alginate, alginate propylene glycol esters; etc. may be present.

As synthesized water-soluble polymers, vinyl base polymers such as a polyvinyl alcohol, polyvinylmethyl ether, polyvinylpyrrolidone, carboxyvinyl polymer (Carbopol), alkyl modified carboxyvinyl polymer; polyoxyethylene base polymers such as polyethylene glycol 2000, 4000, 6000; acryl base polymers such as polyoxyethylene polyoxypropylene copolymer base polymer, sodium polyacrylate, polyethylene acrylate, polyacryl amide, polyethylene imine, cationic polymer, etc. may be present.

As inorganic water-soluble polymers, bentonite, laponite, hectonite, inorganic silicic acid, etc. may be present.

As the thickeners, carragheenin, karaya gum, tragacanth gum, carob gum, quince seed (Marumero), caseine, dextrin, gelatin, sodium pectinate, sodium alginate, methylcellulose, ethylcellulose, CMC, hydroxyethylcellulose, hydroxypropylcellulose, PVA, PVM, PVP, sodium polyacrylate, carboxyvinyl polymer, loqust bean gum, guar gum, tamarind gum, dialkyldimethyl ammonium sulfate cellulose, xanthane gum, aluminum magnesium silicate, bentonite, hectonite, etc. may be present.

As powder components, inorganic powders such as talc, kaolin, mica, sericite, dolomite, phlogopite, synthetic mica, lepidolite, biotite, vermiculite, magnesium carbonate, calcium carbonate, aluminum silicate, barium silicate, calcium silicate, magnesium silicate, strontium silicate, metal salts of tungstenic acid, magnesium, silica, zeolite, barium sulfate, sintered calcium sulfate (sintered gypsum), calcium phosphate, fluorapatite, hydroxyapatite, ceramic powder, metal soap (zinc myristate, calcium palmitate, ammonium stearate), boronitride; organic powders such as polyamide resin powder (nylon powder), polyethylene powder, methyl polymethacrylate powder, polystyrene powder, styrene and acrylic acid copolymer resin powder, benzoguanamin resin powder, polytetrafluoroethylene powder, cellulose powder;

Moisturizers such as polyethylene glycol, propylene glycol, glycerin, 1,3-butylene glycol, hexylene glycol, xylitol, sorbitol, maltitol, chondroitin sulfuric acid, hyaluronic acid, mucoitin sulfuric acid, caronic acid, atelocollagenous protein, cholesteryl-12-hydroxystearate, sodium lactate, gallates, dl-pyrrolidone carboxylates, short chain soluble collagenous protein, diglycerin (EO) PO adducts, Hestnut rose (Rosa roxburghii) extract, Yarrow (Achillea millefolium) extract, Sweet clover (Melilotus officinalis) extract, tranexamic acid may be present.

Anti-UV radiation agents may be present, examples being activating agents such as royal jelly, photosensitive agents or cholesterol derivatives.

Carriers may be water or oil based. Suitable oils include avocado oil, primrose oil, turtle oil, corn oil, mink oil, olive oil, rapeseed oil, egg oil, sesame oil, persic oil, wheat germ oil, sasanqua oil, castor oil, linseed oil, safflower oil, cottonseed oil, perilla oil, soybean oil, peanut oil, teaseed oil, kaya oil, rice bran oil, China wood oil, Japanese wood oil, jojoba oil, germ oil, triglycerin, glycerin trioctanate, glycerin triisopalmitate, etc. Fats may be present such as cacao butter, coconut oil, horse fat, hydrogenated coconut oil, palm oil, beef tallow, sheep fat, hydrogenated tallow, palm kernal oil, hog fat, beef bone fat, Japan wax nut oil, hydrogenated oil, beef hoof fat, Japan wax, hydrogenated castor oil, etc., as the waxes, beeswax, candelilla wax, cotton wax, carnauba wax, bayberry wax, insect wax, spermaceti, montan wax, rice bran wax, lanolin, kapok wax, lanolin acetate, liquid lanolin, sugarcane wax, isopropyl lanolin fatty acid, hexyl laurate, hydrogenated lanolin, jojoba wax, hard lanolin, shellac wax, POE lanolin alcohol ether, POE lanolin alcohol acetate, POE cholesterol ether, polyethylene glycol lanolin fatty acid, POE hydrated lanolin alcohol ether, etc., and as the hydrocarbon oils, liquid paraffin, ozokerite, squalane, pristane, paraffin, ceresine, squalene, vaseline, microcrystalline wax.

As a higher fatty acid, for example, lauric acid, myristic acid, palmitic acid, stearic acid, behenic acid, oleic acid, 12-hydroxystearic acid, undecylic acid, toluic acid, isostearic acid, linolic acid, linoleic acid, eicosapentaenoic acid (EPA), docosahexaenoic acid (DHA), etc. may be present.

As a higher alcohol, for example, linear alcohols such as lauryl alcohol, cetyl alcohol, stearyl alcohol, behenyl alcohol, myristyl alcohol, oleyl alcohol, cetostearyl alcohol; and branched alcohols such as monostearyl glycerin ether (batyl alcohol), 2-decyltetradecinol, lanolin alcohol, cholesterol, phytosterol, hexyldodecanol, isostearyl alcohol, octyldecanol, etc. may be present.

As synthetic ester oils, isopropyl myristate, cetyl octanate, octyldodecyl myristate, isopropyl palmitate, butyl stearate, hexyl laurate, myristyl myristate, decyl oleate, hexyldecyl dimethyloctanate, cetyl lactate, myristyl lactate, lanolin acetate, isocetyl stearate, isocetyl isostearate, cholesteryl 12-hydroxystearate, ethylene glycol di-2-ethylhexylate, dipentaerythritol fatty acid ester, N-alkylglycol monoisostearate, neopentylglycol dicaproate, diisostearyl malate, glycerin di-2-heptyl undecanoate, trimethylopropane tri-2-ethylhexylate, trimethylopropane triisostearate, pentanerythritol tetra-2-ethylhexylate, glycerin tri-2-ethylhexylate, trimethylopropane triisostearate, cetyl-2-ethylhexanoate, 2-ethylhexyl palmitate, glycerin trimyristate, glyceride tri-2-heptylundecanoate, castor oil fatty acid methyl ester, oil oleate, cetostearyl alcohol, acetoglyceride, 2-heptylundecyl palmitate, diisobutyl adipate, N-lauroyl-L-glutamate-2-octyl dodecyl ester, di-2-heptylundecyl adipate, ethyl laurate, di-2-ethylhexyl sebatate, 2-hexyldecyl myristate, 2-hexyldecyl palmitate, 2-hexyldecyl adipate, diisopropyl sebatate, 2-ethylhexyl succinate, ethyl acetate, butyl acetate, amyl acetate, triethyl citrate, etc. may be present.

The composition may take a variety of forms, including a firming serum in which a firming component is present. This may be whey protein and a plant extract. The concentration may be about 6% to about 9% of the composition.

The compositions will be presented as liquids and typically a carrier/solvent system is used. The skin composition may be an aqueous solution, emulsion cream, oil, gel, ointment, aerosol, facial cleanser, pack, mask, bath preparation and the like.

The invention includes a method of reducing wrinkles on the skin, comprising applying a composition as disclosed herein to the skin, especially to the skin of a smoker.

Use of the composition gives good absorption into the skin, a good application feel in addition to control and significantly reduce wrinkles.

In order that the invention may be well understood it will now be illustrated with reference to the following example.

### EXAMPLE

A composition is made by mixing the components of each phase and then adding the subsequent phase to the preceding one in the sequence shown, the quantity of each ingredient or mixture being shown in grams.
**PHASE A:**
   aqua (58.327)
   glycerin, sodium pca urea,threhalose, polyquaternium-51, sodium hyaluronate (10.000)
**PHASE B:**
   cyclopentasiloxane, peg-8, phospholipids, polyphosphorylcholine glycol acrylate (3.750)
   petrolatum, phospholipids, polyphosphorylcholine glycol acrylate (2.875)
   dimethicone/vinyl dimethicone crosspolymer, peg-8, cyclopentasiloxane, phospholipids, polyphosphorylcholine glycol acrylate (2.550)
   hydroxypropyl starch phosphate, carbomer, acrylates/c10-30 alkyl acrylate crosspolymer, acrylates/vinyl isodecanoate crosspolymer, xanthan gum, ceratonia siliqua gum, cyamopsis tetragonoloba gum (1.785)
   hydrogenated polyisobutene, butylene glycol, cetearyl alcohol, phospholipids, polyphosphorylcholine glycol acrylate (1.469)
   hydrogenated polyisbobutene, peg-8, phospholipids, hydrogenated butylene/ethylene/styrene copolymer, ydrogenated ethylene/propylene/styrene copolymer, polyphosphorylcholine glycol acrylate, sodium carbomer (1.250)
**PHASE C:**
   juglans regia (6.000)
   tripeptide-1 (3.000)
   fagus sylvatica (2.000)
   mannitol, pisum sativum, histidine hcl, arginine, acetyl tyrosine, pyridoxine, khaya senegalensis, nicotinamide adenine dinucleotide, disodium succinate, aspartic acid (1.500)
   maltodextrin, malpighia glabra (1.000)
   mica, superoxide dismutase, pyrus malus, cochlearia armoracia, retynyl palmitate, tocopheryl acetate, phospholipids, butylene glycol (1.000)
   phospholipids, tocopheryl acetate, ascorbyl palmitate (1.000)
   phospholipids, tocopheryl acetate, retinylpalmitate (1.000)
**PHASE D:**
   phenoxyethanol (0.624)
   methylparaben (0.164)
   diazolidinyl urea (0.090)
   ethylparaben (0.071)
   propylparaben (0.015)
   butylparaben (0.013)
   isobutylparaben (0.007)
**PHASE E:**
   menthyl lactate,
   ppg-26-butheth-26,
   peg-40 hydrogenated castor oil (0.500)
   parfum (0.010)

The composition according to this recipe can take a variety of forms. In one particular form the composition is a light cream which is applied twice daily. The user will notice an effect after a twenty-eight day period. In another form the composition includes extracts of firming agents such as thymus serpyllum or lupinus albus or whey protein to form a moisture firming serum.

## Claims

1. A skin composition comprising in combination:
• anti-ageing component
• anti-stress component, and
• anti-oxidant component

2. A composition according to Claim 1, wherein each component comprises a mixture of agents having their respective function.

3. A composition according to Claim 1 or 2, wherein the anti-ageing component comprises a blend of plant extracts.

4. A composition according to Claim 3, wherein the extracts are of Walnut and Beech.

5. A composition according to Claim 3 or 4, wherein the extracts are derived from buds and shoots of the plants.

6. A composition according to any preceding Claim, wherein the anti-ageing component makes up from about 10% to about 12% by weight of the composition.

7. A composition according to any preceding Claim, wherein the anti-stress component comprises a mixture of Vitamins.

8. A composition according to Claim 7, wherein the anti-stress component makes up from about 2% to about 3% by weight of the composition.

9. A composition according to any preceding Claim, wherein the anti-oxidant comprises a stimulant for the glutathione system such as a protein and/or an enzyme.

10. A composition according to Claim 9, wherein the protein or enzyme is presented in micro-encapsulated form.

11. A composition according to Claim 9 or 10, wherein the concentration of anti-oxidant component is from about 2% to about 3% by weight of the composition.

12. A composition according to any preceding Claim, including a firming component.

13. A composition according to Claim 12, wherein the firming component comprises whey protein and a plant extract.

14. A method according to Claim 13, wherein the plant extract is thymus serpyllum or lupinus albus.

15. A composition according to Claim 12, 13 or 14, wherein the firming component makes up from about 6% to about 9% of the composition.

16. A method of reducing the wrinkles on a skin, the method comprising applying a composition according to any preceding Claim, to the skin.

17. A method according to Claim 16, comprising applying a composition in the form of a light cream twice daily.

18. A method according to Claim 16, wherein the composition includes a firming composition which is applied to the skin as a serum.

19. A method according to any of Claims 16 to 18, wherein the skin is that of a smoker.
